(19) Europäisches Patentamt
European Patent Office
Office européen des brevets

(11) Publication number: 0 048 044 A1

(12) EUROPEAN PATENT APPLICATION

(21) Application number: 81200928.0

(22) Date of filing: 25.08.81

(51) Int. Cl.[3]: C 07 D 295/14
A 61 K 31/495

(30) Priority: 12.09.80 NL 8005132

(43) Date of publication of application:
24.03.82 Bulletin 82/12

(84) Designated Contracting States:
AT BE CH DE FR GB IT LI LU NL SE

(71) Applicant: DUPHAR INTERNATIONAL RESEARCH B.V
C.J. van Houtenlaan 36
1381 CP Weesp(NL)

(72) Inventor: Haeck, Hans Heinz
c/o OCTROOIBUREAU ZOAN B.V. Apollolaan 151
NL-1077 AR Amsterdam(NL)

(72) Inventor: Hillen, Feddo Cornelius
c/o OCTROOIBUREAU ZOAN B.V. Apollolaan 151
NL-1077 AR Amsterdam(NL)

(74) Representative: Muis, Maarten, Drs. et al,
OCTROOIBUREAU ZOAN B.V. Apollolaan 151
NL-1077 AR Amsterdam(NL)

# (54) Phenyl piperazine derivatives having antiagressive activity.

(57) It has been found that the group of new phenyl piperazine derivatives of formula 3 of the formula sheet and the salts thereof have a strong antiagressive activity. The compounds can be prepared in a manner known for analogous compounds and be processed to the usual compositions.

EP 0 048 044 A1

Croydon Printing Company Ltd.

# Phenyl piperazine derivatives having antiagressive activity

The invention relates to new phenyl piperazine derivatives, to the preparation of these compounds, and to compositions comprising at least one of these compounds as an active substance.

Various phenyl piperazine derivatives with or without pharmacological properties are known.

From Indian J. Appl. Chem. (1972), 35, pp. 129-130 (which article is referred in Chem. Abstr. 81, 120577 n), the preparation is known of five compounds of formula 1 of the formula sheet, in which R is the phenyl group, p-chlorophenyl group, p-fluorophenyl group, p-methoxyphenyl group or 3,5-dimethoxyphenyl group.

These compounds have been prepared in the scope of an investigation into new anthelmintics.

Furthermore, an article in J. Med. Chem. 9, (1966), pp. 153-155 relates to compounds of formula 2, in which $R_1$ may be the phenyl group or 4-chlorophenyl group, $R_2$ is, for example, hydrogen, methyl, ethyl, propyl, tert. butyl, phenyl or substituted phenyl, and n is 1 or 2.

The compounds have been tested for several activities and in most of the cases proved to have no activity.

It has now been found that phenyl piperazine derivatives of formula 3 of the formula sheet, in which $R_3$ is a trifluoromethyl group or a halogen atom, $R_4$ is an alkyl group or an alkoxy group having 1-3 C-atoms, a cycloalkoxy group having 5-7 C-atoms, trifluoromethyl, vinyl, nitrile, phenyl, halogen-, alkyl-, alkoxy- or trifluoromethyl-substituted phenyl, a thienyl group, pyridyl group or pyrimidyl group, or a group $-NR_5R_6$, in which $R_5$ and $R_6$ each are a hydrogen atom, an alkyl group having 1 or 2 C-atoms, or a vinyl group, and A is the group $-CH_2-$, $-CH_2CH_2-$, $CH_3-CH-CH_2-$ or $-CH_2-CH-CH_3$ and the salts thereof, have a strong antiagressive activity. Some of the compounds belonging to this

group are in addition readily analgetically active. In formula 3, $R_4$ is preferably amino, methyl, ethoxy, trifluoromethyl or a phenyl group substituted by chlorine, fluorine, methyl or methoxy.

The antiagressive activity of the compounds was measured in a test suitable for this purpose on isolated mice (Advances in Pharmacol. 5, (1967), 79). In this test, male albino mice were kept isolated for a period of 4 weeks and were then selected for the test on the basis of the presence of fighting behaviour. The selection criterion is the occurrence of 3 or more fighting periods within 3 minutes after a mouse not kept isolated was placed in the cage of the mouse kept isolated.

The compounds to be investigated were administered orally to the selected mice. Per dosis 5 mice were used. Sixty minutes after the administration of the compound to be investigated the animals were again evaluated for fighting behaviour. The compound to be investigated is inactive in the dosis administered when this time also 3 or more fighting periods were observed within 3 minutes after a mouse not kept isolated was placed in the cage of an isolated mouse. From the results obtained the $ED_{50}$-value in mg of active substance per kg of body weight was calculated.

The compounds according to the invention have an $ED_{50}$-value which is smaller than 10 mg/kg and for most compounds the $ED_{50}$-value is 1-5 mg/kg.

Due to the strong antiagressive activity and the absence of undesired side effects, such as sympatholytic, dopaminolytic, muscle relaxing and sedative properties, the compounds are excellently suitable for administration in the treatment of intrapunitive and extrapunitive behaviour and overt agressive behaviour in man and animal.

For medical use in men is to be considered first of all the control of agressive symptoms in psychiatric illnesses and serious forms of psychopathologic agression.

As a possibility of application in the veterinary field are to be considered above all those forms of agression which occur in transporting agricultural domestic animals and the mixing of groups of these animals.

The quantity, frequency and mode of administration may differ for each individual case, also dependent on the nature and the severity of the disturbances. Generally, a dose from 5-500 mg, and preferably from 25-150 mg daily will be suitable for application in men.

For veterinary applications the dose is preferably from 0.1-10 mg/kg of body weight.

The active compounds according to the invention and their salts can be processed according to known standard methods to compositions such as pills, tablets, coated tablets, capsules, powders, injection liquids and the like while using the conventional auxiliary substances such as solid and liquid carrier materials.

As examples of pharmaceutically acceptable acids with which the compounds according to the invention can form salts may be mentioned hydrochloric acid, sulphuric acid, nitric acid, citric acid, fumaric acid, maleic acid, tartaric acid, methanesulfonic acid, benzoic acid and the like.

The compounds of formula 3 and their salts can be prepared according to methods suitable for the synthesis of analogous compounds. The invention therefore also relates to the preparation of the new compounds and the salts thereof.

Dependent on the meaning of $R_4$, the compounds of formula 3 can be obtained according to at least one of the following methods of preparation.

By reaction of a compound of the general formula 4 of the formula sheet with a compound of the formula $Cl-A-CO-NH-CO-R_4$. This reaction is preferably carried out in an inert solvent such as ethanol or acetonitrile at a temperature between 20°C and the boiling point of the solvent used, in the presence of an acid binder.

Compounds of formula 3 according to the invention, in which $R_4$ is an amino group or an alkoxy group, can also be obtained by reacting a compound of formula 5 with ammonia, alkylamine, methanol, or ethanol. This reaction is preferably carried out in an organic solvent such as ether, tetrahydrofuran, benzene, toluene, or methylene chloride, at a temperature between 0°C and the boiling point of the

solvent used (see United States Patent Specification 3,155,700 and J. Org. Chem. 28, (1963), p. 1805).

Furthermore, compounds of formula 3, in which $R_4$ is for example, $NH_2$, alkyl, alkoxy or phenyl, can also be obtained by reaction of a compound of formula 6 with a compound of the formula $NH_2$-CO-$R_4$. This reaction is preferably carried out by heating the starting substances without solvent at 100°C for a few hours.

The compounds of formula 3 can furthermore be obtained by reaction of a compound of formula 7 with a compound of the formula $NH_2$-A-CO-NH-CO-$R_4$. This conversion is preferably carried out in a solvent such as butanol, in the presence of an acid-binding agent such as $K_2CO_3$, at temperatures between room temperature and the boiling point of the solvent (see British Patent Specification 943,739).

Finally, the compounds of formula 3 can be obtained in similar reaction circumstances by reaction of a compound of formula 8 with a compound of formula 9 (see Coll. Czech. Chem. Comm. 6, (1934), 211).

The invention will be described in greater detail with reference to the examples below.

EXAMPLE I

N-{3-[4-(3-chlorophenyl)-1-piperazinyl]propionyl}urea.

12 Mmol (2,4 g) of 3-chlorophenyl piperazine and 12 mmol (1.8 g) of 3-chloropropionyl urea were dissolved together in 20 ml of absolute ethanol. After having added 12 mmol (1.0 g) of sodium bicarbonate to this solution, the reaction mixture was refluxed for 2 hours. The reaction mixture was then evaporated to dryness under reduced pressure. Water was added to the residue while stirring vigorously, after which the precipitate formed was sucked off. The material obtained in this manner was then recrystallized from ethanol, after which the title compound was obtained having a melting-point of 190.5-192°C.

In an analogous manner the following compounds were prepared:

1) N-{3-[4-(3-trifluoromethylphenyl)-1-piperazinyl]propionyl urea} (melting-point 185-187°C) from 3-trifluoromethylphenyl piperazine and 3-chloropropionyl urea.

2) N-{3-[4-(3-trifluoromethylphenyl)-1-piperazinyl]-2-methylpropionyl} urea (melting-point 165-166°C) from 3-trifluoromethylphenyl piperazine and 3-chloro-2-methylpropionyl urea.

3) N-{3-[4-(3-trifluoromethylphenyl)-1-piperazinyl]-3-methylpropionyl} urea (melting-point 160-161°C) from 3-trifluoromethylphenyl piperazine and crotonyl urea.

4) N-{3-[4-(3-trifluoromethylphenyl)-1-piperazinyl] propionyl -N'-methyl} urea (melting-point 142,5-143,5°C) from 3-trifluoromethylphenyl piperazine and N-(3-chloropropionyl)-N'-methyl urea.

5) N-{2-[4-(3-trifluoromethylphenyl)-1-piperazinyl] acetyl} urea (melting-point 153.5-154.5°C), from 3-trifluoromethylphenyl piperazine and 2-chloroacetyl urea.

## EXAMPLE II

N-{3-[4-(3-trifluoromethylphenyl)-1-piperazinyl] propionyl} acetamide

20 Mmol (4.6 g) of 3-trifluoromethylphenyl piperazine, 20 mmol (3 g) of N-acetyl-3-chloropropionamide and 20 mmol (1.7 g) of sodium bicarbonate were refluxed together in 20 ml of acetonitrile for 15 minutes. The undissolved material was then sucked off and the filtrate was evaporated under reduced pressure. The residue was taken up in 50 ml of methylene chloride and, after drying over potassium carbonate, the solution was evaporated under reduced pressure and the residue was recrystallized from ethyl acetate/petroleum ether. The crystallisate was then purified chromatographically over silica gel with methylene chloride/methanol (10:1) as an eluent. After evaporating the solvents, the free base thus obtained was recrystallized from ether/petroleum ether, the title compound being obtained having a melting-point of 71-74°C.

Analogous to the above-described method of preparation, the following compound was prepared:

N-{3-[4-(3-trifluoromethylphenyl)-1-piperazinyl] propionyl} benzamide (melting-point 80-82.5°C) from 3-trifluoromethylphenyl piperazine and N-(3-chloropropionyl)benzamide.

## EXAMPLE III

Ethyl N-{3-[4-(3-trifluoromethylphenyl)-1-piperazinyl] propionyl}carbamate

40 Mmol (7,16 g) of ethyl N-3-chloropropionyl carbamate, 40 mmol (9.28 g) of 3-trifluoromethylphenyl piperazine and 20 mmol (2.76 g) of potassium carbonate were refluxed together in 4Q ml of acetonitrile for 6 hours. The reaction mixture was then evaporated to dryness under reduced pressure, taken up in 100 ml of methylene chloride and dried on 5.6 g of potassium carbonate. The dried solution was then evaporated under reduced pressure and the residue was recrystallized twice from ethyl acetate. The title compound obtained in this manner had a melting-point of 78-79°C.

Analogous to the above method of preparation the following compound was prepared:

Cyclohexyl N-{3-[4-(3-trifluoromethylphenyl)-1-piperazinyl] propionyl}carbamate.HCl (melting-point 198-200°C; decomposition), from 3-trifluoromethylphenyl piperazine and cyclohexyl N-3-chloropropionyl carbamate.

EXAMPLE IV

N-{3-[4-(3-trifluoromethylphenyl)-1-piperazinyl]propionyl} urea.HCl

24 Mmol (5.5 g) of 3-trifluoromethylphenyl piperazine, 24 mmol (3.6 g) of 3-chloropropionyl urea and 24 mmol (2 g) of sodium bicarbonate were refluxed together in 40 ml of absolute ethanol for 5 hours. The reaction mixture was then evaporated to dryness under reduced pressure and the residue was taken up in 2 N hydrochloric acid. The precipitate formed was sucked off. The sucked off material was recrystallized from water after which the title compound was obtained having a melting-point of 212-213°C.

EXAMPLE V

N-{3-[4-(3-trifluoromethylphenyl)-1-piperazinyl]propionyl} urea

10 Mmol (0.6 g) of urea and 10 mmol (3.41 g) of 3-[4-(3-trifluoromethylphenyl)-1-piperazinyl]propionyl chloride were homogenized together and then heated at 100-110°C for 1 hour. After cooling, 2 N NaOH was added to the reaction mixture until pH 8. The undissolved material was sucked off and then purified chromatographically over silica gel with methylene chloride/methanol (100:1) as an eluent.

After evaporating the solvents, the free base thus obtained was recrystallized from ethyl acetate, the title compound being obtained having a melting-point of 185-187.5°C.

EXAMPLE VI

Ethyl N-{3-[4-(3-trifluoromethylphenyl)-1-piperazinyl]propionyl}carbamate

5.5 Mmol (0.47 ml) of oxalyl chloride were added dropwise to a solution of 5.5 mmol (1.6 g) of 3-[4-(3--trifluoromethylphenyl)-1-piperazinyl]propionamide in 50 ml of 1,2-dichloroethane while stirring vigorously and under an atmosphere of nitrogen, the temperature being kept between 5 and 10°C. The reaction mixture was then refluxed for 16 hours. After cooling, the precipitate present was sucked off and the filtrate was evaporated to dryness under reduced pressure. The residue was taken up in 25 ml of aboslute ethanol and, after leaving to stand at room temperature for 2 hours, the solution was again evaporated under reduced pressure. The material thus obtained was stirred with 2 N sodium hydroxide and then extracted with methylene chloride. The organic layer was dried on potassium carbonate and, after evaporating the solvent, the residue was purified chromatographically over silica gel with ethyl acetate as an eluent. After evaporating the solvent, the free base thus obtained was recrystallized from ethyl acetate. The title compound obtained in this manner had a melting-point of 78-79°C.

CLAIMS:

1. Compounds of formula 3 of the formula sheet and salts thereof with pharmaceutically acceptable acids, in which formula $R_3$ is a trifluoromethyl group or a halogen atom,

$R_4$ is an alkyl group or an alkoxy group having 1-3 C-atoms, a cycloalkoxy group having 5-7 C-atoms, trifluoromethyl, vinyl, nitrile, phenyl, halogen-, alkyl-, alkoxy-, or trifluoromethyl-substituted phenyl, a thienyl group, a pyridyl group or pyrimidyl group, or a group $-NR_5R_6$, wherein $R_5$ and $R_6$ each represent a hydrogen atom, an alkyl group having 1 or 2 C-atoms or a vinyl group, and

A is the group $-CH_2-$, $-CH_2-CH_2-$, $CH_3-CH-CH_2-$ or $-CH_2-CH-CH_3$.

2. N-{3-[4-(3-trifluoromethylphenyl)-1-piperazinyl]propionyl}urea and salts thereof with pharmaceutically acceptable acids.

3. N-{3-[4-(3-chlorophenyl)-1-piperazinyl]propionyl}urea and salts thereof with pharmaceutically acceptable acids.

4. N-{3-[4-(3-trifluoromethylphenyl)-1-piperazinyl]propionyl}acetamide and salts thereof with pharmaceutically acceptable acids.

5. N-{3-[4-(3-trifluoromethylphenyl)-1-piperazinyl]-2-methyl propionyl}urea and salts thereof with pharmaceutically acceptable acids.

6. Ethyl N-{3-[4-(3-trifluoromethylphenyl)-1-piperazinyl] propionyl}carbamate and salts thereof with pharmaceutically acceptable acids.

7. N-{3-[4-(3-trifluoromethylphenyl)-1-piperazinyl]-3-methyl propionyl}urea and salts thereof with pharmaceutically acceptable acids.

8. N-{3-[4-(3-trifluoromethylphenyl)-1-piperazinyl]propionyl}-N'-methyl urea and salts thereof with pharmaceutically acceptable acids.

9. N-{3-[4-(3-trifluoromethylphenyl)-1-piperazinyl]propionyl}benzamide and salts thereof with pharmaceutically acceptable acids.

10. Pharmaceutical compositions, characterized in that they comprise as an active substance at least one compound of formula 3 of the formula sheet, wherein $R_3$, $R_4$ and A have the meanings given in Claim 1, or a salt thereof with a pharmaceutically acceptable acid.

11. A method of preparing pharmaceutical compositions, characterized in that a compound of Claim 1 is brought into a form suitable for administration.

12. A method of preparing pharmaceutically active phenyl piperazine derivatives, characterized in that antiagressively active compounds of formula 3 of the formula sheet, wherein $R_3$, $R_4$ and A have the meanings given in Claim 1, and salts thereof with pharmaceutically acceptable acids are prepared in a manner known for the synthesis of analogous compounds.

13. A method as claimed in claim 12, characterized in that a compound of formula 4 of the formula sheet is converted with a compound of the formula Cl-A-CO-NH-CO-$R_4$, in which formulae $R_3$, $R_4$ and A have the meanings given in Claim 1.

14. A method as claimed in claim 12, characterized in that a compound of formula 3 is prepared, in which $R_4$ is an amino group, an alkylamino group, a methoxy group or an ethoxy group, and $R_3$ and A have the meanings given in Claim 1, by converting a compound of formula 5 with ammonia, alkylamine, methanol or ethanol.

15. A method as claimed in claim 12, characterized in that a compound of formula 6 of the formula sheet is converted with a compound of the formula $NH_2$-CO-$R_4$, in which formulae $R_3$, $R_4$ and A have the meanings given in Claim 1.

16. A method as claimed in claim 12, characterized in that a compound of formula 7 of the formula sheet is converted with a compound of the formula $NH_2$-A-CO-NH-CO-$R_4$ in which $R_3$, $R_4$ and A have the meanings given in Claim 1.

17. A method as claimed in claim 12, characterized in that a compound of formula 8 is converted with a compound of formula 9 of the formula sheet, in which formulae $R_3$, $R_4$ and A have the meanings given in Claim 1.

18. A method of controlling intrapunitive and extrapunitive behaviour and overt agressive behaviour in man or animal, characterized in that an active quantity of a compound of formula 3, in which $R_3$, $R_4$ and A have the meanings given in claim 1, or a salt thereof with a pharmaceutically acceptable acid, is administered.

CLAIMS FOR AUSTRIA

1. A method of preparing pharmaceutically active phenyl piperazine derivatives, characterized in that antiagressively active compounds of formula 3 of the formula sheet, wherein $R_3$ is a trifluoromethyl group or a halogen atom,

$R_4$ is an alkyl group or an alkoxy group having 1-3 C- atoms, a cycloalkoxy group having 5-7 C-atoms, trifluoromethyl, vinyl, nitrile, phenyl, halogen-, alkyl-, or trifluoromethyl-substituted phenyl, a thienyl group, a pyridyl group or pyrimidyl group, or a group $-NR_5R_6$, wherein $R_5$ and $R_6$ each represent a hydrogen atom, an alkyl group having 1 or 2 C-atoms, or a vinyl group, and

A is the group $-CH_2-$, $-CH_2-CH_2-$, $CH_3-CH-CH_2-$ or $-CH_2-CH-CH_3$,

and salts thereof with pharmaceutically acceptable acids are prepared in a manner known for the synthesis of analogous compounds.

2. A method as claimed in Claim 1, characterized in that a compound of formula 4 of the formula sheet is converted with a compound of the formula $Cl-A-CO-NH-CO-R_4$, in which formulae $R_3$, $R_4$ and A have the meanings given in Claim 1.

3. A method as claimed in Claim 1, characterized in that a compound of formula 3 is prepared, in which $R_4$ is an amino group, an alkylamino group, a methoxy group or an ethoxy group, and $R_3$ and A have the meanings given in Claim 1, by converting a compound of formula 5 with ammonia, alkyl amine, methanol or ethanol.

4. A method as claimed in Claim 1, characterized in that a compound of formula 6 of the formula sheet is converted with a compound of the formula $NH_2-CO-R_4$, in which formulae $R_3$, $R_4$ and A have the meanings given in Claim 1.

5. A method as claimed in claim 1, characterized in that a compound of formula 7 of the formula sheet is converted with a compound of the formula $NH_2-A-CO-NH-CO-R_4$ in which $R_3$, $R_4$ and A have the meanings given in Claim 1.

6. A method as claimed in Claim 1, characterized in that a compound of formula 8 is converted with a compound of formula 9 of the formula sheet, in which formulae $R_3$, $R_4$ and A have the meanings given in Claim 1.

1/1



DIR 0314

FORMULA-SHEET

1.

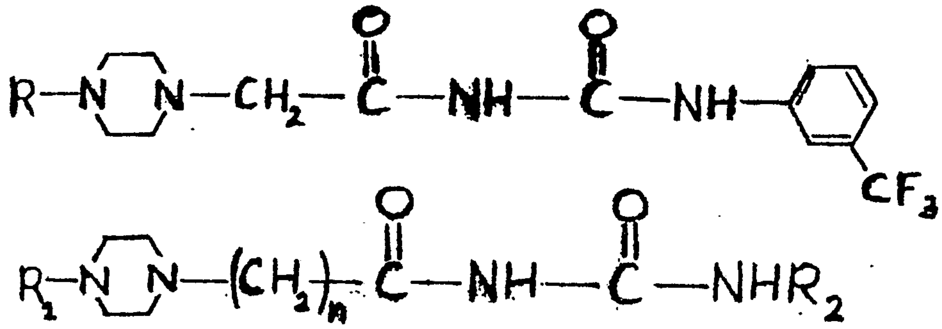

2.

3.

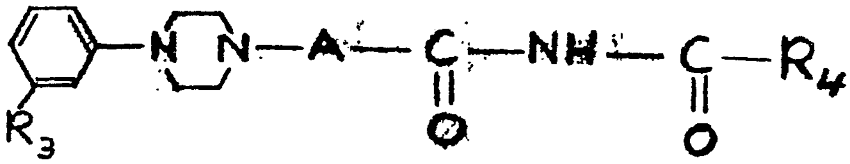

4.

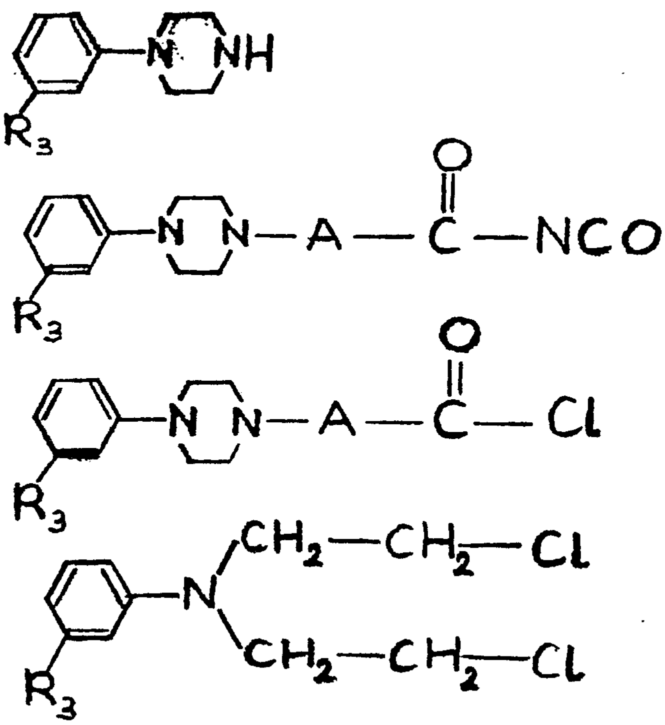

5.

6.

7.

8.

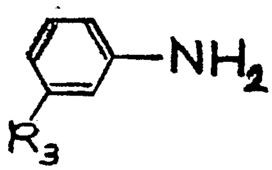

9.

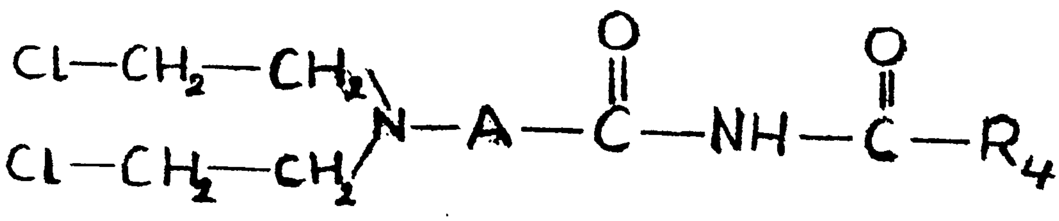

DUPHAR INTERNATIONAL RESEARCH B.V.

0048044

European Patent Office

# EUROPEAN SEARCH REPORT

Application number
EP 81 20 0928

| Category | Citation of document with indication, where appropriate, of relevant passages | Relevant to claim | CLASSIFICATION OF THE APPLICATION (Int. Cl.3) |
|---|---|---|---|
| | FR - A - 2 209 227 (PARCOR)<br>* Claims *<br>-- | | C 07 C 295/14<br>A 61 K 31/495 |
| D | JOURNAL OF MEDICINAL CHEMISTRY, vol. 9, january 1966<br>American Chemical Society, Easton, Pennsylvania<br>M. VERDERAME: Synthesis of 1,4-Disubstituted Piperazines. I[1]", pages 153-154<br>* Pages 153-154<br>-- | | TECHNICAL FIELDS SEARCHED (Int. Cl.3)<br>C 07 D 295/14 |
| D | CHEMICAL ABSTRACTS, vol. 81, no. 19, 11th November 1974, abstract 120577n, page 534<br>Columbus, Ohio, US<br>S.S. TIWARI et al.: "Search for new anthelmintics. IV. Synthesis of acetyl-N-(3-(trifluoromethyl) phenyl)ureide of substituted mono-arylpiperazines"<br>& Indian J. Appl. Chem. 1972, 35(4-6), 129-30<br>* Abstract *<br>---- | | |

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

X The present search report has been drawn up for all claims

| Place of search | Date of completion of the search | Examiner |
|---|---|---|
| The Hague | 16-12-1981 | MOREAU |